# EUROPEAN PATENT SPECIFICATION

(11) **EP 2 094 095 B1**
(45) Date of publication and mention of the grant of the patent: **06.07.2016**
(21) Application number: 07835415.6
(22) Date of filing: 09.11.2007
(51) Int. Cl.: A23C 3/08, A23L 3/3445, A23L 2/44, A23L 2/54, A23L 3/3409, C02F 1/78, A23L 3/358, A61L 2/20

(54) **OZONE TREATMENT OF LIQUID FOODSTUFF**
OZONBEHANDLUNG VON FLÜSSIGEM NAHRUNGSMITTEL
TRAITEMENT À L'OZONE D'ALIMENTS LIQUIDES

(30) Priority: 30.11.2006 SE 0602584
(43) Date of publication of application: 02.09.2009
(73) Proprietor: Pastair AB, 227 30 Lund (SE)
(72) Inventor: SJÖHOLM, Johan, 244 78 Lund (SE)
(74) Representative: KIPA AB
(86) International application number: PCT/SE2007/050831
(87) International publication number: WO 2008/066470

(56) References cited:
- WO-A-99/14163
- WO-A1-02/089612
- DE-A1- 3 325 568
- DE-A1- 4 426 648
- US-A- 2 901 356
- US-A- 4 767 528
- US-A1- 2005 186 310

## Description

### Technical field

The present invention relates to a method and an apparatus for treating liquid foodstuffs with ozone.

### Background of the invention

In particular milk is contaminated with microorganisms, and in particular with spores and spore forming bacteria being shelf-live destroying to the milk or being pathogenic to the consumer, which bacteria require some type of sterilization including pasteurization in order to produce a product that can be stored for more than 24 hrs.

Milk having a high content of spores cannot be used in the production of cheeses, either hard or soft cheeses. Besides the problem in production of cheese, this will also means economical consequences for the farmers delivering the milk, as they can loose up to 25% of the price depending on quality classification..

Pasteurization - the process of heating food for the purpose of killing harmful organisms such as bacteria, viruses, protozoa, molds, and yeasts. The process was named after its inventor, French scientist Louis Pasteur. The first pasteurization test was completed by Pasteur and Claude Bernard on April 20, 1862.

Unlike sterilization, pasteurization is not intended to kill all microorganisms in the food, as compared to appertization, invented by Nicolas François Appert. Instead, pasteurization aims to achieve a "log reduction" in the number of viable organisms, reducing their number so they are unlikely to cause disease (assuming the pasteurized product is refrigerated and consumed before its expiration date). Commercial scale sterilization of food is not common, because it adversely affects the taste and quality of the product.

The dairy industry in the United States has a long history of producing a safe, wholesome, and convenient beverage for consumers. This enviable record is the result of the industry's ability to adapt its processing, packaging, and handling of this complex product to meet food safety requirements and consumer needs.

Milk is a complex biological fluid. It possesses many functional properties and characteristics; but it is milk's flavour and nutritional value that sets it apart from other beverages.

Unfortunately, the same biological attributes that set milk apart from other beverages also make it an excellent media for microbiological growth. This microbiological growth can be in the form of spoilage bacteria and pathogens. It is through proper heat treatment, or pasteurization that these organisms are destroyed.

It is known to process fruit juices using high pressure and gamma radiation to destroy microorganisms.

In 1864 Louis Pasteur discovered that bacteria could be destroyed by heat. It soon became common practice to pasteurize milk in vats as the benefits of safe, and longer shelf-life milk were recognized.

In the 1930's, the High Temperature Short Time (HTST) system of pasteurizing milk was perfected. Soon the standard of pasteurizing milk through an HTST became industry norm.

The parameters for pasteurization in the United States fall under The Pasteurized Milk Ordinance (PMO), a cooperative effort of industry and state regulatory agencies in conjunction with the Food and Drug Administration. For white fluid milk the time-temperature relationship for HTST processed milk is a minimum of 71,6°C (161°F) for at least 15 seconds.

Fluid milk processing plants have traditionally pasteurized milk at higher temperatures for longer periods of time as an extra safety factor. (Historically fluid milk is pasteurized in the 74,4 - 76,6°C (166-170°F) range for 20-25 seconds.) Pasteurizing milk at this time/temperature ratio typically gives a clean slightly cooked flavour with a 5-15 days shelf life.

More recently, under the recommendations of FDA, and its concerns regarding food safety, many fluid milk plants are increasing their HTST pasteurization temperatures to 80-81,1°C (176-178°F).

In recent years, new technology has been developed that increases the shelf life of fluid dairy products. Studies have shown that pasteurizing milk at Higher Heat Shorter Time (HHST) ratios [also referred to as Ultra-Pasteurized (UP) or Extended Shelf Life (ESL)] will provide a safe product while increasing the shelf life of milk to 50 days or more.

A second parameter has been added to the PMO for the pasteurization of milk as Ultra-Pasteurized (UP) milk. The time/temperature requirement for UP milk is at least 137,7°C (280 "F) for at least 2 seconds. Most plants in the United States that are processing UP milk are pasteurizing in the 137,7-143,3°C (280-290°F) range for 2-4 seconds.

However, it should be noted that while increasing the pasteurization temperature of milk increases its shelf life, it also amplifies the "cooked" flavour in the product, as well as a brownish colour, probably due to caramellization. While this cooked flavour is not objectionable to most consumers it does create a different flavour profile when compared to standard HTST milk.

Nutritionally, there is no difference between HTST and UP milk. Bacteriologically, both products are safe, but UP milk will keep longer in refrigerated storage and can be given a longer code date.

Organoleptically, UP milk usually has a more intense "cooked" flavour. The flavour differences, however, are not objectionable to most consumers and are becoming more subtle than in the past.

UHT and UP are distributed ambient, while HTST is distributed refrigerated.

While having little effect on shelf life, studies have shown that the "cooked" flavour is more pronounced with the higher processing temperatures. The net result is that the difference between the flavour of HTST milk and UP milk is becoming less pronounced. Regardless the choice. HTST or UP, consumers can feel confident the milk they drink will be safe, nutritious, and pleasant tasting.

General pasteurization takes place by heating the product during a very short period as indicated above, and under certain circumstances an ultra high temperature is used to provide for a long-term storability, so called UHT milk. Normal heat treatment provides for milk which has a storability of about 7-14 days after production and filling, while UHT milk can be stored up to 6 months or longer. Sterilization may take place in so called clean room environment or closed filling equipment as Tetra Pak® Aseptic, i.e., an environment where all air added is filtered free from any microorganism carried, the equipment is kept clean and free of microorganisms, and the personal is dressed in such a way as not introducing microorganisms therein, in many cases the treatment is made automatic without any presence of operating personal.

While pasteurization conditions effectively eliminate potential pathogenic microorganisms, it is not sufficient to inactivate the thermoresistant spores in milk. The term sterilization refers to the complete elimination of all microorganisms. The food industry uses the more realistic term "commercial sterilization", a product is not necessarily free of all microorganisms, but those that survive the sterilization process are unlikely to grow during storage and cause product spoilage.

Some examples of food products processed with UHT are:
liquid products - milk, juices, cream, yoghurt, wine, salad dressings foods with discrete particles - baby foods; tomato products; fruits and vegetables juices; soups
larger particles - stews

The difficulties with UHT is seen in the sterility conditions; the complexity of equipment and plant that are needed to maintain sterile atmosphere between processing and packaging (packaging materials, pipework, tanks, pumps) together with higher skilled operators. and that sterility must be maintained through aseptic packaging.

Heat stable lipases or proteases can lead to flavour deterioration, age gelation of the milk over time. There is also a more pronounced cooked flavour to UHT milk.

The HTST pasteurization standard was designed to achieve a 5-log reduction (0.00001 times the original) in the number of viable microorganisms in milk. This is considered adequate for destroying almost all yeasts, mold, and common spoilage bacteria and also to ensure adequate destruction of common pathogenic heat-resistant organisms (including particularly Mycobacterium tuberculosis, which causes tuberculosis and Coxiella burnetii, which causes Q fever).

### Alternative pasteurization standards and raw milk

In addition to the standard HTST and UHT pasteurization standards, there are other lesser-known pasteurization techniques. The first technique, called "batch pasteurization", involves heating large batches of milk to a lower temperature, typically 68 °C (155 °F). The other technique is called higher-heat/shorter time (HHST), and it lies somewhere between HTST and UHT in terms of time and temperature. Pasteurization causes some irreversible and some temporary denaturization of the proteins in milk.

Advocates of raw milk maintain, correctly, that some components survive in milk that has not been pasteurized. Specifically, raw milk contains immunoglobulins and the enzymes lipase and phosphatase, which are inactivated by heat. Raw milk also contains vitamin B6 of which up to 20% may be lost on heat treatment. It is also claimed to contain beneficial bacteria which aid digestion and boost immunity.

Commercial distribution of packaged raw milk is not allowed in most US states.
Some doctors (and some raw milk advocates) acknowledge that certain people should not drink raw milk, including pregnant or breast-feeding mothers, those undergoing immunosuppression treatment for cancer, organ transplant or autoimmune diseases, and those who are immunocompromised due to diseases like AIDS.

In fact, some doctors suggest that babies and breast-feeding mothers avoid all but UHT pasteurized dairy products.

In Africa, it is common to boil milk whenever it is harvested. This intense heating greatly changes the flavor of milk, which the people in Africa are accustomed to.

Thus HTST and UHT methods are associated with change in taste and flavour of the milk treated, as well as it is associated with high investment costs with regard to equipment to carry out the pasteurization or UHT treatment.

Today the farmers meet problems in keeping the bacterial count down in the raw milk due to new ensiling methods. A cold pasteurization that provides a high bactericidal effect (90 % killed) prior to a heat pasteurization could mean that fresh milk delivered from the dairies may have 10 times lower bacterial count than today.

Cold pasteurization may thus provide completely new possibilities to the food industry, primarily by reducing costs, increase quality and increase productivity.

Cold pasteurization can increase the quality of the product by avoiding high temperature treatment or reduced the spore count prior to pasteurization. By means of cold pasteurization new functional food and health products can reach the market.

The saving using cold pasteurization will be 1 million kW compared to regular pasteurization, which is an environmentally positive effect.

It is generally recognized that if the raw milk should contain a low bacterial count, then a longer shelf life will be obtained of the fresh milk. The aim is to be able to obtain a 3 to 4 week storability in refrigerator while maintaining good taste, in contrast to HTST and UHT with regard to taste and colour.

For this purpose it has meant that microfiltration equipment has been developed wherein the milk is filtrated. Hereby the bacterial count can be kept down and thereby the storability can be increased. Such microfiltration equipments are voluminous and expensive.

Other processes developed to increase storability is an electronic radiation treatment, high pressure plants etc. The common feature of these processes is that the investment costs as well as maintenance costs are relatively high, 10-15 million SEK.

Carbon dioxide has been used in small quantities as an "add back" in fresh milk. Thus it has been showed that an addition of 200 - 400 ppm of CO₂ increased storability to the double.

The problem of using CO₂ is that the package material needs to be gastight and the distribution needs to take place under refrigerated conditions.

The cold pasteurization proposed by the present invention is not any expensive process, but the investment level can be kept down to below 1-2 million SEK treating at least 50 million litres of fluid and year, and simultaneously the maintenance costs will be low.

Thus preservation of milk is a great problem.

WO 96/24386 discloses a method for treating body fluids, including milk and blood with ozone, whereby the fluid is atomized prior to ozone treatment in order to afford a faster ozone to fluid reaction.

It is apparent that such a method cannot be used in a dairy where very large volumes of milk shall be treated.

DE-A-3 325 568 discloses an apparatus for ozone treatment of liquids whereby a layer of ozone is contained above a layer of liquid. No real contact area by the interface between the two layers is thus present.

US 2,901,356 discloses a method for preserving liquid food, wherein the water content of he liquid food is reduced by 50-60%. The concentrate is thereafter sterilized by treating the liquid food with a gas containing ozone (approximately 1 L ozonized air per 1 kg food). The ozone is removed and thereafter the temperature of the concentrated and sterilized liquid is stepwise lowered in a plurality of separate cooling stages of 5 °C at a time to between 1-8 °C. Each cooling step takes less than 1 minute and the holding time at each reduced temperature is two hours. Below 8°C the concentrate is lowered 1°C for each step, resting one hour at each temperature until -1°C. Thereafter the temperature is increased to 20 °C again using a reverse procedure. The concentrated liquid thus treated has an increased resistance against spoilage.

DE4426648 discloses a method for treating food products and animal feeds including eggs, egg products, meat and sausage meat, milk and dairy products, vegetables, vegetables and fruit juices, cocoa and cocoa products, almonds, nuts, cereals or cereal products, products from materials of animal origin such as animal meals, extracts of organs, bone parts, bone meal or tissue parts, tea, coffee, tobacco and tobacco products, comprising fumigating the products with ozone-containing air or ozone-containing oxygen. The treatment is carried out with ozone concentrations of 1-280 µg/ml (0.02-15 wt.%). The contact time between the ozone containing gas and the product is e.g. for 5 seconds to 5 hrs..

US 4.767,528 discloses a drinking water purifying apparatus comprising an ozone generator, and means for contacting ozone with water, whereby the apparatus further comprises a means for reducing the ozone concentration, which latter ozone gas is used for sterilization. The disclosure denotes extremely long contact times between water and ozone gas amounting to up to 30 minutes or more. The amount of ozone dispersed in the water amounts to about 2 milligrams per litre.

US 2005/0186310 A1 discloses a process for treating foods under alternating atmospheres, whereby an ozone gas is fed to a food processing system under pressure, the pressure is hold under a certain time period, and subsequently feeding an inert gas to remove the residual amounts of ozone. The pressure used is 50 to 2500 psig. There is no teaching that ambient or lower pressure can be used to sterilize food products such as liquid food products. Further, the disclosure indicates a pretty long pressure holding time which means a long contact time period.

### Summary of the present invention

The present invention aims at solving the problem of pasteurizing fluids, in particular milk, at low temperatures using a gaseous medium.

The present invention thus aims to solve the problems of preserving in particular milk, and is in particular applicable on fresh, raw milk, which may contain a fairly amount of microorganisms.

### Detailed description of the present invention

In particular the present invention relates to a method for inhibiting bacterial growth according to claim 1.

In a preferred embodiment the temperature of the biological liquid medium at the treatment is ambient temperature.

In a preferred embodiment the temperature of the biological liquid medium when being milk is 4 to 20°C, being the ambient storage and transport temperature of milk.

In a preferred embodiment the amount of ozone added is at least 3, 6, 24 or 100 ppm.

In a preferred embodiment the amount of ozone added is 1 to 10 ppm.

In a preferred embodiment the ozone is distributed into the liquid medium via a perforated inlet device at a pressure of less than 1 bar.

In a preferred embodiment the ozone is distributed over en enlarged surface area to the liquid medium.

In a preferred embodiment the size of the ozone gas bubbles has a diameter of 0.5 to 5 µm.

In a preferred embodiment the ozone gas bubbles have a diameter of 1 to 2 µm.

In a preferred embodiment the dwell time is less than 30 sec. more preferably less than 20 sec. still more preferably less than 10 sec., still more preferably less than 5 sec.

In a preferred embodiment the ozone amount is 3 to 6 ppm, and the dwell time is 6 to 7 seconds.

In a preferred embodiment the degassing for eliminating excess ozone is carried out by applying a subpressure or vacuum.

In a preferred embodiment the degassing for eliminating excess ozone is carried out by applying a vacuum, preferably at a reduced pressure of at least 10 mmHg (1.333 kPa).

In a preferred embodiment the degassing for eliminating excess ozone is carried out by adding finely distributed nitrogen and/or carbon dioxide gas, while applying a subpressure.

In a preferred embodiment a pre-treatment step is carried out prior to the treatment of the actual biological liquid according to one or more of claims 1-10, by having water passing the different steps of the method, while ozone treating such water.

In a preferred embodiment a post-treatment step is carried out subsequent to a cleansing operation of an apparatus in which the method of the present invention has been carried out according to claims 1-10, for the treatment of the actual biological liquid, by having water passing the different steps of the method, while ozone treating such water.

A further aspect of the invention relates to an apparatus for carrying out the method disclosed above, according to claim 13.

In a preferred embodiment the gas injection unit filter is made of a disposable material, as fluids treated may be very sensitive to contaminants.

In a preferred embodiment the injection site where the gas injection unit filter is introduced into a production line is designed to provide for the gas being introduced to become solved or mixed into the fluid to be treated.

In a preferred embodiment the gas injection unit filter is present of a number of porous, ozone distributing fingers extending across a liquid flow passway

The gas injection unit filter should preferably be made of a disposable material, as fluids treated may be very sensitive to contaminants. Thus any gas injection unit should be replaced daily, or even more frequently if there should have been a production stop.

The gas injection unit consists of, besides the gas injection unit filter of a fluid meter and a controlling function to provide for the adequate amount of gas being provided. In a preferred embodiment of the invention the gas injection filter is arranged in such a way that only a small slot is available to the liquid to pass the filter, whereby the distance between the filter and a surrounding tube wall is only some flew millimetres, such as 2 to 5 mm, such as 2, 3, 4, or 5 mm.

The injection site where the gas injection unit filter is introduced into a production line is important and should be designed in such a way that the gas is immediately solved or mixed into the fluid to be treated.

The liquid to be treated should preferably have a temperature of less than 20°C. The pressure of the inlet ozone should be around or below 1 bar in order to produce the optimal gas bubbles into the passing liquid. The flow of ozone through the gas injection unit filter is adapted to the pressure of the gas as well as to the flow of surrounding bypassing liquid to be treated to provide for a ozone amount of at most 100 ppm, as indicated above.

Hereby it may be advantageous to use a non-return valve to avoid reflush of ozone into storage bins.

The dwell time unit consists of a peristaltic pump unit in which the biological fluid will not become to much macerated.

In the Nordic countries there are produced 10 x 10⁹ litres of milk each year. Of this amount different products are produced such as cheese, yoghurt, sour milk, cottage cheese, cream and milk of different qualities (normally different fat contents as a base).

When producing cheese the spore count of the ingoing milk is of greatest importance. If the spore count is high the productivity declines linearly with the number of spores. This is one main reason for paying the dairy farmers less per litre, i.e., a high spore count-less income.

Using the present system any environmental impact will become reduced as the daily transport of milk will be reduced. Further, the saving using cold pasteurization will be 1 million kW compared to regular pasteurization, which is an environmentally positive effect.
Furthermore, the production of cheese will increase. The disposal of out-dated milk is a great environmental load. By using the present invention the out-dating quantities are expected to become much lower. The quality of the cheese will become improved, as no high temperature pasteurization is carried out.

Gas injection is facilitated by means of filter needles being placed in the production flow. By using disposable filter needles the hygienic conditions can be kept at a high standard.

To obtain an adequate killing of bacteria and spores the contact time between gas and fluid must be guaranteed. This contact time is very precise when it comes to milk, as too short contact time will give an inadequate killing, and too long contact time will provide taste changes to the milk.

The contact time is facilitated by means of a dwell time unit, in the form of a peristaltic pump, whereby the tube forming part of the pump is separated into cells. Hereby the pump will guarantee that the dwell time in each cell is constant and maintained. From a qualitative point of view the tube needs to be replaced ever so often. The total dewll time includes degassing time.

Besides milk, soft drinks, soy milk, oat milk, liquid egg products (e.g. pancake suspension), water etc can be cold pasteurized using the present method and apparatus. Furthermore, the killing of bacteria in blood is possible (Arch. Med. Res. 37 (2006) 425-435, V. A. Bocci, Scientific and Medical Aspects of Ozone Therapy, State of the Art).

The term "raw, fresh milk" means herein harvested milk that has not been subject to any treatment, but optionally cooling during storage and transport.

The term "microorganism" used herein shall mean any microorganism including bacteria, virus, fungi or yeast, thus also including spores of such a microorganisms.

In a test made on so called mini milk - a pasteurized milk having a fat content of 0.5 %, consisting of standard milk from which the cream has been separated and raw, fresh milk - untreated, non-homogenized milk having a fat content of about 3.9 % 750 ml samples of each milk were subjected to an ozone treatment in accordance with the table below, whereby the ozone was in each case finely distributed throughout the whole passage area. The result of the testing is shown in the table 1 below.

**Table 1**

| No | Sample | Product | Volume | Time (min) | Total amount (cfu/ml) |
|---|---|---|---|---|---|
| 1 | Rinsing | | | | |
| 2 | Reference | Mini | 750 ml | 2.5 | 4400 |
| 3 | 35 ppm | Mini | 750 ml | 2.5 | 9200 |
| 4 | 200 ppm | Mini | 750 ml | 5 | 200 |
| 5 | Taste ref. | Mini | 3000 ml | 10 | |
| 6 | Rinsing | Fresh milk | | | |
| 7 | Reference | Fresh milk | 750 ml | 2.5 | 23000 |
| 8 | 35 ppm | Fresh milk | 750 ml | 2.5 | 29000 |
| 9 | 200 ppm | Fresh milk | 750 ml | 5 | 2000 |
| 10 | Taste ref. | Fresh milk | 3000 ml | 10 | |

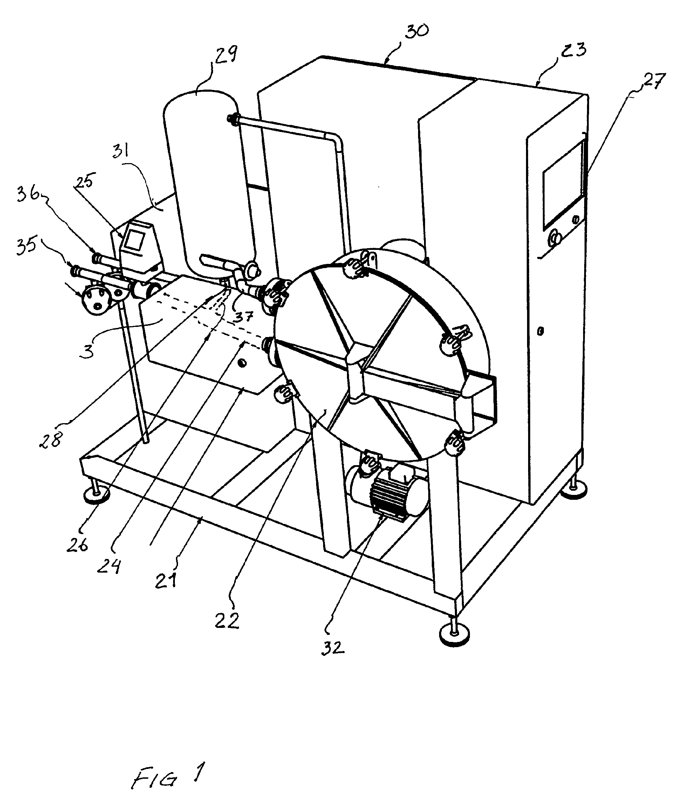

As mentioned above one aspect of the invention relates to an equipment for carrying out the method. One embodiment of the equipment is described in the attached drawing, wherein
FIG. 1 shows a general diagram of a layout of such an equipment, and
FIG. 2 shows a porous means used in the equipment,
FIG. 3 shows a preferred embodiment of a porous gas injector device; and

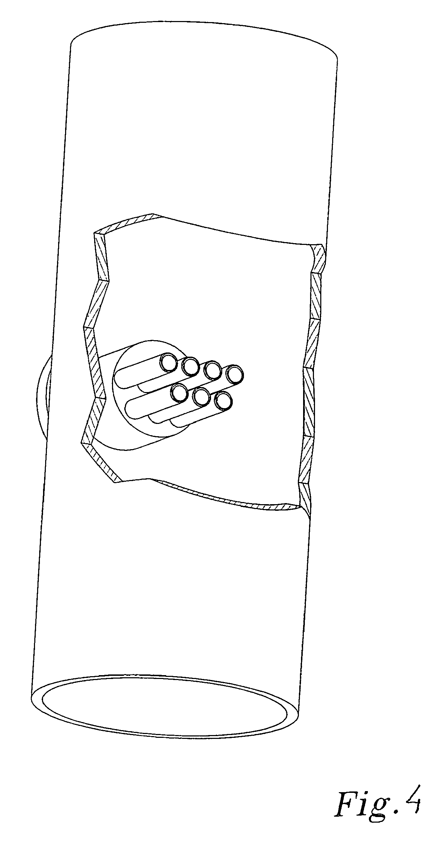

FIG. 4 shows the injector device of FIG. 3 placed in a reaction tube.

A suitable equipment or apparatus for subjecting milk for an ozone treatment consists of a tripod 21 onto which ozone holder cell 22 is arranged. Further there is an electrical cabinet 23 maintaining electrical control 27 and supply units (not shown). In front of the ozone holder cell 22 there is an ozone product inlet 24 comprising a ozone injector 26. An ozone generator 31 is connected to the ozone generator outlet 28. A supply vessel (not shown) is connected to a product inlet 35 to feed a liquid such as milk to the system. The ozone injector 26 is placed in a gassing station 3 arranged in the product feed line and subsequent to the gassing station 3 there is a tube system to transfer the liquid into the ozone holder cell 22 being a peristaltic pump. The ozone injector 26, where ozone gas is introduced, comprises one or more porous means 4 having each a volume of about 2 to 25 cm³ and provided with pores having a size of 2 µm, whereby the ozone to be added will be added throughout the whole area of milk to pass by. The milk is then drawn by means of the peristaltic pump 22 via a ozone holder cell product outlet 37 to a degassing station 29 wherein the milk is degassed, optionally while adding nitrogen and/or carbon dioxide to aid in the removal of surplus of ozone dissolved in the milk. The liquid is then finally removed from the ozone treatment apparatus via a product outlet 36. Such aiding gas is supplied via a conduit from a gas source supplying said nitrogen and/or carbon dioxide. The peristaltic pump 22 will provide for a dwell time amounting to 3 to 10 seconds or more. Controlling the rate of the peristaltic pump 22 can easily control this dwell time. The peristaltic pump 22 will thereby take care of the whole transport of milk from the supply vessel to the degassing station 29. After the degassing station 29, which normally operates under some vacuum or subpressure supplied by means of a vacuum pump 32, the milk will be packed in suitable containers, and is passed to an HTST pasteurisation (71"C) prior to being packed, such as into cardboard packages, or bottles, or other types of distribution vessels. The milk may be packed and further treated, and distributed for further processing, as well. The vacuum applied at the degassing station is such that a substantially complete removal of ozone contained in the milk is removed.

The ozone injector 26 is arranged in such a way that it can be readily removed for cleansing and/or exchange. The microbiology status is important handling foodstuffs in liquid form.

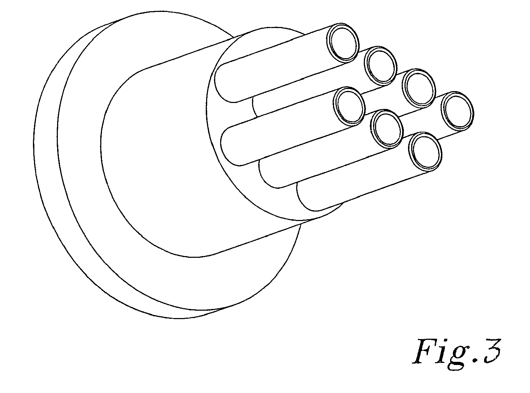

The ozone injector may, preferably take the form of a multiple injector, shown in FIG. 3, comprising a number of perforated "fingers" 26A through which the ozone is introduced. The fingers 26A are applied perpendicular, or substantially perpendicular to the liquid flow, and whereby the distance between the fingers fulfils the requirements concerning distance between wall and ozone distributor to be able to treat all the liquid volume passing the ozone distributor. The fingers can be placed in a line, or as shown in a zigzag pattern having five fingers in one line and three fingers in a second line. The fingers are thereby placed in a holder being connected to the ozone producing unit. In case the liquid comprises solids, such as when an orange juice is treated the solids may optionally build up onto the fingers. Thereby, a vibration movement is applied onto the holder to provide a shaking movement removing the solids build-up.

These fingers further have a pore size of 1 to 2 µm through which the ozone is introduced into the liquid.

The vacuum or subpressure applied will be at least 3.33kPa (25 mmHg), preferably 6.66kPa (50 mmHg), preferably at least 9.99 kPa (75 mmHg), more preferably at least 16;67 kPa (125 mmHg), still more preferably at least 23,33 kPa (175 mmHg), most preferably at least 29,98 kPa (225 mmHg). The basic step is to ventilate the ozone out of the milk using a slight subpressure, which may be less than 1.33 kPa (10 mmHg).

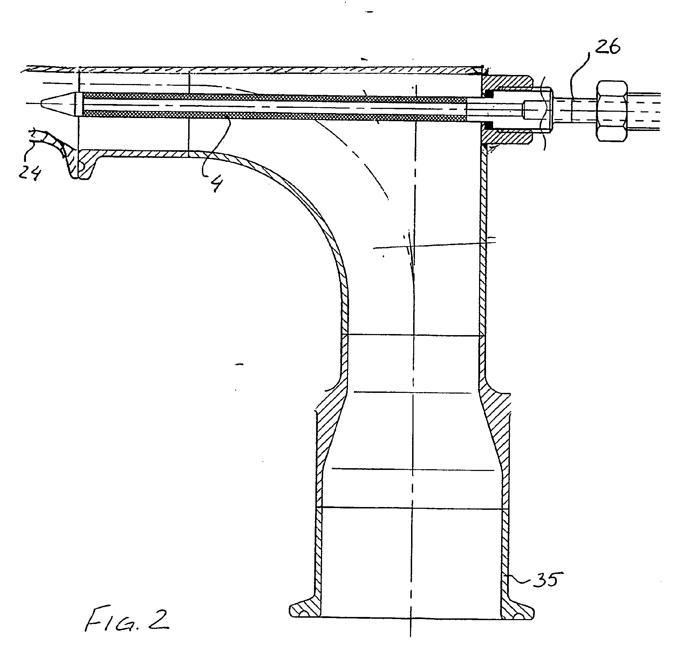

The porous means 4 of the ozone injector 26 present in the gassing station 3 is shown in detail in FIG. 2. As the porous means is designed longitudinal the milk will pass the porous structure during a relatively long pathway, leading to an efficient mixing in of the gas. In order to further increase this efficiency, the conduit in which the porous means 4 is present can be narrowed to reduce the volume around the porous means, thereby increasing the possibility of a gassing over the whole cross section, i.e., increasing the active volume meeting the flow of extremely small ozone gas bubbles hitting the liquid flow preferably in a direction perpendicular thereto.

In a further test pasteurized standard milk was tested. The contact time between milk and ozone was set at below 10 seconds. The dosage of ozone was less than 10 ppm. A taste panel could not determine any off-taste.

The test showed a killing of 0.4 log (59%). As the milk was pasteurised the amount of free fatty acids are greater, and thereby the sensitivity to oxidation.

The killing of the microorganisms is apparently independent of a concentration of ozone in this test. This is probably due to the fact that the test was carried out using pasteurised milk. Ozone kills spore forming microorganisms, and probably thereby some types more easily. The killing effect is better than that obtained using common pasteurisation, 0.4 log is remarkably good having an already pasteurised milk to start with. The result obtained is shown in the table 2 below.

**Table 2**

| gO₃/m³ | cfu/ml | % killing |
|---|---|---|
| 0 | 717 | |
| 3.5 | 365 | |
| 5 | 250 | 59,11 |
| 20 | 450 | |
| 50 | 480 | |
| 125 | 263 | |

## Claims

1. A method for inhibiting bacterial growth in a biological liquid media, said method comprising passing a biological liquid medium past a finely divided gas stream containing ozone which is distributed into the liquid medium via a porous means providing ozone to the biological liquid medium in an amount of at least 1 ppm of liquid medium treated, after which the liquid medium is passed on to a dwell time unit wherein in said dwell time unit, during a dwell time of 1 minute or less, complete mixing between liquid and ozone, followed by a degassing step to eliminate excess of ozone dissolved in the liquid medium is provided, **characterized in that** the dwell time unit is a tubular mixer having restricted mixing chambers, wherein the tubular mixer is a peristaltic pump, whereby the tube forming part of the pump is separated into cells.

2. A method according to claim 1, wherein the temperature of the biological liquid medium being treated is ambient temperature or less, more preferably the temperature is 4 to 15 °C.

3. A method according to claim 1, wherein the amount of ozone added is at least 3, 6, 24, 50, 75 or 100 ppm, more preferably the ozone added is 3 to 10 ppm of liquid medium treated.

4. A method according to claim 1, wherein the ozone is distributed into the liquid medium via a perforated inlet device at a pressure of less than 1 bar.

5. A method according to claim 3, wherein the size of the ozone gas bubbles has a diameter of 1 to 2 µm, more preferably 0.5 to 5 µm.

6. A method according to claim 1, wherein the dwell time is less than 30 sec. more preferably less than 20 sec. still more preferably less than 10 sec., still more preferably less than 5 sec.

7. A method according to claim 1, wherein the ozone amount is 3 to 6 ppm, and the dwell time is 6 to 7 seconds.

8. A method according to claim 1, wherein the degassing for eliminating excess ozone is carried out by applying a subpressure or vacuum.

9. A method according to claim 1, wherein the degassing for eliminating excess ozone is carried out by applying a vacuum, preferably at a reduced pressure of at least 1.333 kPa (10 mm Hg).

10. A method according to claim 1, wherein the degassing for eliminating excess ozone is carried out by adding finely distributed nitrogen and/or carbon dioxide gas, while applying a subpressure.

11. A method according to claim 1, wherein a pre-treatment step is carried out prior to the treatment of the actual biological liquid according to one or more of claims 1-10, by having water passing the different steps of the method, while ozone treating such water.

12. A method according to claim 1, wherein a post-treatment step is carried out subsequent to a cleansing operation of an apparatus in which the method of the present invention has been carried out according to claims 1-10, for the treatment of the actual biological liquid, by having water passing the different steps of the method, while ozone treating such water.

13. An apparatus for carrying out the method according to claims 1-12, which apparatus comprises a gas injection unit, a dwell time unit, and a degassing unit, whereby an ozone source is provided, preferably an ozone producing unit for an intermittent or continuous production of ozone, whereby a control unit is provided to control the process, said gas injection unit further comprising a fluid meter and a controlling function that provides for an adequate amount of gas **characterized in that** the dwell time unit is a tubular mixer having restricted mixing chambers, wherein the tubular mixer is a peristaltic pump, whereby the tube forming part of the pump is separated into cells.

14. Apparatus according to claim 13, wherein the gas injection unit is provided with one or more porous, ozone distributing fingers extending across a liquid flow passway.

## Patentansprüche

1. Verfahren zur Hemmung von bakteriellem Wachstum in einem biologischen flüssigen Medium, wobei das Verfahren das Durchlaufen eines biologischen flüssigen Mediums vorbei an einem feinzerteilten Gasstrom umfasst, welcher Ozon enthält, das über ein poröses Mittel in dem flüssigen Medium verteilt wird, wodurch dem biologischen flüssigen Medium Ozon in einer Menge von mindestens 1 ppm von behandeltem flüssigen Medium zugeführt wird, wonach das flüssige Medium an eine Verweilzeit-Einheit übermittelt wird, wobei in dieser Verweilzeit-Einheit im Laufe einer Verweilzeit von einer Minute oder weniger eine vollständige Vermischung zwischen Flüssigkeit und Ozon gefolgt von einem Entgasungs-Schritt vorgesehen ist, um den Überschuss an Ozon abzuscheiden, welches in dem flüssigen Medium gelöst ist, **dadurch gekennzeichnet, dass** es sich bei der Verweilzeit-Einheit um einen Rohrmischer handelt, welcher begrenzte Mischkammern aufweist, wobei es sich bei dem Rohrmischer um eine Peristaltikpumpe handelt, wodurch der Schlauch, welcher einen Teil der Pumpe bildet, in Zellen aufgeteilt wird.

2. Verfahren gemäß Anspruch 1, wobei die Temperatur des behandelten biologischen flüssigen Mediums Raumtemperatur oder weniger beträgt, und stärker bevorzugt die Temperatur von 4 bis 15°C hat.

3. Verfahren gemäß Anspruch 1, wobei die hinzugegebene Ozonmenge mindestens 3, 6, 24, 50, 75 oder 100 ppm beträgt, und stärker bevorzugt das hinzugegebene Ozon 3 bis 10 ppm des behandelten flüssigen Mediums beträgt.

4. Verfahren gemäß Anspruch 1, wobei das Ozon über eine perforierte Einlassvorrichtung unter einem Druck von weniger als 1 bar in dem flüssigen Medium verteilt wird.

5. Verfahren gemäß Anspruch 3, wobei die Größe der Ozon-Gasblasen einem Durchmesser von 1 bis 2 µm, und stärker bevorzugt 0,5 bis 5 µm, entspricht.

6. Verfahren gemäß Anspruch 1, wobei die Verweilzeit weniger als 30 Sekunden, stärker bevorzugt weniger als 20 Sekunden, noch stärker bevorzugt weniger als 10 Sekunden und noch stärker bevorzugt weniger als 5 Sekunden beträgt.

7. Verfahren gemäß Anspruch 1, wobei die Ozonmenge 3 bis 6 ppm und die Verweilzeit 6 bis 7 Sekunden beträgt.

8. Verfahren gemäß Anspruch 1, wobei die Entgasung zum Zwecke der Abscheidung von überschüssigem Ozon durch Anwendung eines Unterdrucks oder Vakuums ausgeführt wird.

9. Verfahren gemäß Anspruch 1, wobei die Entgasung zum Zwecke der Abscheidung von überschüssigem Ozon durch Anwendung eines Vakuums ausgeführt wird, vorzugsweise unter einem geminderten Druck von mindestens 1,333 kPa (10 mm Hg).

10. Verfahren gemäß Anspruch 1, wobei die Entgasung zum Zwecke der Abscheidung von überschüssigem Ozon durch Zugabe von feinzerteiltem Stickstoff und/oder Kohlendioxidgas unter Anwendung eines Unterdrucks ausgeführt wird.

11. Verfahren gemäß Anspruch 1, wobei vor der Behandlung der eigentlichen biologischen Flüssigkeit gemäß einem oder mehreren der Ansprüche 1 bis 10 ein Vorbehandlungsschritt ausgeführt wird, und zwar indem man Wasser die verschieden Stufen des Verfahrens durchlaufen lässt, während dieses Wasser einer Ozonbehandlung unterzogen wird.

12. Verfahren gemäß Anspruch 1, wobei anschließend an einen Reinigungsvorgang eines Geräts, in welchem das Verfahren der vorliegenden Erfindung gemäß Ansprüchen 1 bis 10 ausgeführt wurde, ein Nachbehandlungsschritt ausgeführt wird, um die eigentliche biologische Flüssigkeit zu behandeln, und zwar indem man Wasser die verschiedenen Stufen des Verfahrens durchlaufen lässt, während dieses Wasser einer Ozonbehandlung unterzogen wird.

13. Gerät zur Ausführung des Verfahrens gemäß Ansprüchen 1 bis 12, wobei das Gerät eine Gasinjektions-Einheit, eine Verweilzeit-Einheit sowie eine EntgasungsEinheit umfasst, wodurch eine Ozonquelle vorgesehen wird, vorzugsweise eine Ozonerzeugungs-Einheit für eine intermittierende oder kontinuierliche Erzeugung von Ozon, wodurch eine Steuereinheit vorgesehen wird, um den Prozess zu steuern, wobei die Gasinjektions-Einheit ferner einen Flüssigkeitsmesser und eine Steuerungsfunktion umfasst, durch welche eine adäquate Menge an Gas bereitgestellt wird, **dadurch gekennzeichnet, dass** es sich bei der Verweilzeit-Einheit um einen Rohrmischer handelt, der begrenzte Mischkammern aufweist,
wobei es sich bei dem Rohrmischer um eine Peristaltikpumpe handelt, wodurch der Schlauch, welcher einen Teil der Pumpe bildet, in Zellen aufgeteilt wird.

14. Gerät gemäß Anspruch 13, wobei die Gasinjektions-Einheit mit einem oder mehreren porösen, ozonverteilenden Fingern versehen ist, die sich durch den flüssigen Strömungsdurchgang erstrecken.

## Revendications

1. Procédé destiné à inhiber une croissance bactérienne dans un milieu liquide biologique, ledit procédé comprenant l'étape consistant à faire passer un milieu liquide biologique par un flux gazeux finement divisé contenant de l'ozone qui est distribué dans le milieu liquide par le biais d'un moyen poreux fournissant l'ozone au milieu liquide biologique suivant une quantité d'au moins 1 ppm de milieu liquide traité, après quoi le milieu liquide est transmis vers une unité de temps de maintien dans lequel dans ladite unité de temps de maintien, au cours d'un temps de maintien de 1 minute ou moins, un mélange complet entre le liquide et l'ozone, suivi d'une étape de dégazage pour éliminer l'excès d'ozone dissous dans le milieu liquide est prévu, **caractérisé en ce que** l'unité de temps de maintien est un mélangeur tubulaire ayant des chambres de mélange limitées, dans lequel le mélangeur tubulaire est une pompe péristaltique, grâce à quoi le tube constituant une partie de la pompe est séparé en cellules.

2. Procédé selon la revendication 1, dans lequel la température du milieu biologique liquide qui est traité est la température ambiante ou moins, de façon davantage préférée la température est de 4 à 15 °C.

3. Procédé selon la revendication 1, dans lequel la quantité d'ozone ajouté est d'au moins 3, 6, 24, 50, 75 ou 100 ppm, de façon davantage préférée l'ozone ajouté est de 3 à 10 ppm du milieu liquide traité.

4. Procédé selon la revendication 1, dans lequel l'ozone est distribué dans le milieu liquide par le biais d'un dispositif d'entrée perforé à une pression inférieure à 1 bar.

5. Procédé selon la revendication 3, dans lequel la taille des bulles de gaz d'ozone a un diamètre de 1 à 2 µm, de façon davantage préférée de 0,5 à 5 µm.

6. Procédé selon la revendication 1, dans lequel le temps de maintien est inférieur à 30 s de façon davantage préférée inférieur à 20 s de façon encore davantage préférée inférieur à 5 s.

7. Procédé selon la revendication 1, dans lequel la quantité d'ozone est de 3 à 6 ppm, et le temps de maintien est de 6 à 7 secondes.

8. Procédé selon la revendication 1, dans lequel le dégazage destiné à éliminer l'ozone en excès est réalisé en appliquant une surpression ou un vide.

9. Procédé selon la revendication 1, dans lequel le dégazage destiné à éliminer l'ozone en excès est réalisé en appliquant un vide, de préférence à une pression réduite d'au moins 1,333 kPa (10 mm Hg).

10. Procédé selon la revendication 1, dans lequel le dégazage destiné à éliminer l'ozone en excès est réalisé en ajoutant un gaz d'azote et/ou du dioxyde de carbone finement distribué, tout en appliquant une surpression.

11. Procédé selon la revendication 1, dans lequel une étape de pré-traitement est exécutée avant le traitement du liquide biologique actuel selon une ou plusieurs des revendications 1 à 10, en ayant soumis de l'eau aux différentes étapes du procédé, tout en traitant à l'ozone cette eau.

12. Procédé selon la revendication 1, dans lequel une étape de post-traitement est exécutée après une opération de nettoyage d'un appareil dans lequel le procédé de la prévention a été mis en oeuvre conformément aux revendications 1 à 10, pour le traitement du liquide biologique actuel, en ayant soumis de l'eau aux différentes étapes du procédé, tout en traitant à l'ozone cette eau.

13. Appareil destiné à mettre en oeuvre le procédé selon les revendications 1 à 12, lequel appareil comprend une unité d'injection de gaz, une unité de temps maintien, et une unité de dégazage, grâce à quoi une source d'ozone est prévue, de préférence une unité de production d'ozone pour une production intermittente ou continue d'ozone, grâce à quoi une unité de commande est prévue pour commander le procédé, ladite unité d'injection de gaz comprenant en outre une fonction de mesure et de commande de fluide qui fournit une quantité adéquate de gaz **caractérisé en ce que** l'unité de temps de maintien est un mélangeur tubulaire ayant des chambres de mélange limitées, dans lequel le mélangeur tubulaire est une pompe péristaltique, grâce à quoi le tube constituant une partie de la pompe est séparé en cellules.

14. Appareil selon la revendication 13, dans lequel l'unité d'injection de gaz est dotée d'un ou plusieurs doigts de distribution d'ozone poreux s'étendant en travers d'un passage d'écoulement de liquide.
